(19) Europäisches Patentamt
European Patent Office
Office européen des brevets

(11) Publication number: **0 021 552**
**B1**

# (12) EUROPEAN PATENT SPECIFICATION

(45) Date of publication of patent specification: **26.01.83**

(51) Int. Cl.³: **C 07 D 223/10,**
C 07 D 211/76,
C 07 D 207/26

(21) Application number: **80200701.3**

(22) Date of filing: **06.12.78**

(60) Publication number of the earlier application in accordance with Art. 76 EPC: **0003253**

## (54) Hexahydroazepine, piperidine and pyrrolidine derivatives and processes for preparing them.

(30) Priority: **22.12.77 GB 5337077**
**30.05.78 GB 5337077**

(43) Date of publication of application:
**07.01.81 Bulletin 81/1**

(45) Publication of the grant of the patent:
**26.01.83 Bulletin 83/4**

(84) Designated Contracting States:
**BE CH DE FR IT LU NL SE**

(56) References cited:
**None**

(73) Proprietor: **JOHN WYETH & BROTHER LIMITED**
**Huntercombe Lane South Taplow**
**Maidenhead Berkshire, SL6 0PH (GB)**

(72) Inventor: **Cavalla, John Frederick**
**105 The Grove**
**Isleworth Middlesex (GB)**
Inventor: **White, Alan Chapman**
**5 Sherbourne Drive**
**Windsor, Berkshire (GB)**
Inventor: **Shepherd, Robin Gerald**
**79 Huntercombe Lane North**
**Burnham, Buckinghamshire (GB)**

(74) Representative: **Brown, Keith John Symons et al,**
**c/o John Wyeth & Brother Limited Huntercombe Lane South**
**Taplow Maidenhead, Berkshire SL6 0PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 021 552 B1

Hexahydroazepine, piperidine and pyrrolidine derivatives and processes for preparing them

The invention relates to hexahydroazepine, piperidine, and pyrrolidine derivatives. More particularly the invention relates to certain novel 2-oxo-hexahydroazepine, -piperidine and -pyrrolidine derivatives and to a novel process for preparing the novel derivatives.

Various 3,3-disubstituted hexahydroazepines, -piperidine and -pyrrolidines are known to have pharmacological activity, particularly analgesic activity. For example, analgesic 2-unsubstituted-3,3-disubstituted-hexahydroazepines, such as meptazinol, are disclosed in U.K. Specification No. 1,285,025. Profadol and related 3,3-disubstituted-pyrrolidines are described in J. Med. Chem. 1965, *8*, 316 and Belgian Patent Specification No. 850,777 while myfadol and related 3,3-disubstituted-piperidines are described in J. Med. Chem. 1965, *8*, 313. The known processes for preparing the 3,3-disubstituted-hexahydroazepines, -piperidines and -pyrrolidines are expensive and it is an object of the present invention to provide novel intermediates which may be easily prepared by a novel process from readily available starting materials and which can be converted into the desired 3,3-disubstituted-hexahydroazepines, -piperidines and pyrriolidines such that the overall process for preparing the final products is generally more economic than the known processes.

The novel compounds provided by the invention are 2-oxo-hexahydroazepine, -piperidine and -pyrrolidine derivatives of the general formula (I)

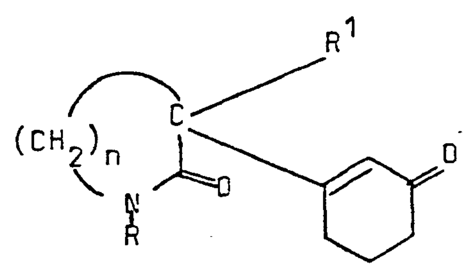
(I)

wherein n is 2,3 or 4, R is hydrogen, lower alkyl or aryl(lower)alkyl, and $R^1$ is hydrogen or lower alkyl.

The term "lower" as used herein means that the radical referred to contains 1 to 6 carbon atoms. The radical preferably contains 1 to 4 carbon atoms. For example when R is lower alkyl, the radical may be, for example, methyl, ethyl, propyl or butyl. Similarly $R^1$ may be, for example, methyl, ethyl, propyl or butyl. When R is aryl(lower)alkyl, the radical is preferably a phenyl(lower)alkyl radical such as phenethyl or benzyl; the phenyl group may be substituted by, for example, one or more substituents such as halogen, alkoxy, trifluoromethyl or other substituents common in medicinal chemistry.

The compounds of general formula (I) may be converted to their aromatised derivatives of general formula (II)

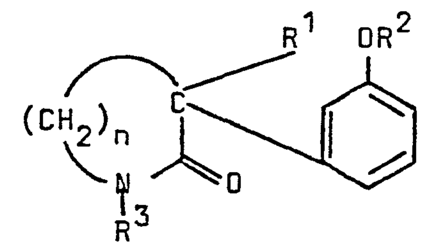
(II)

where n and $R^1$ are as defined above, $R^2$ is hydrogen, lower alkyl or aryl(lower)alkyl and $R^3$ is hydrogen, lower alkyl, aryl(lower)alkyl, lower alkenyl or lower alkynyl.

Where $R^3$ is lower alkenyl or lower alkynyl it is to be understood that the double or triple bond is not in the 1-position of the alkenyl or alkynyl radical; examples of suitable alkenyl and alkynyl radicals are allyl, propargyl, 3,3-dimethylallyl and 1-methyl-2-propynyl.

The compounds of general formula (II) may be prepared by aromatising and optionally O-(lower)alkylating or O-aryl(lower)alkylating the compounds of general formula (I) to give a compound of general formula (II) in which $R^3$ is hydrogen, lower alkyl or aryl(lower)alkyl and, if desired N-alkylating, N-arylalkylating, N-alkenylating or N-alkynylating a compound of general formula (II) in which $R^3$ is hydrogen to give a compound of general formula (II) in which $R^3$ is lower alkyl, aryl(lower)alkyl, lower alkenyl or lower alkynyl.

The process for preparing compounds of general formula (II) from the novel compounds of general formula (I) is claimed and more fully described in European Patent Application No. 78300753.7 (Publication Number 0 003 253), from which the present application is divided.

We have found that the compounds of general formula (I) can be prepared by a novel process from readily starting materials. Accordingly in a further aspect the invention provides a process for

preparing a compound of general formula (I) which comprises reacting a cyclohexene derivative of general formula (IV)

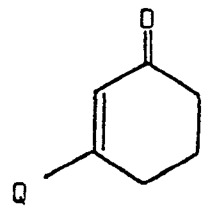

(IV)

where Q is a hydrolysable protecting group such as lower alkoxy (preferably methoxy, ethoxy, or i-propyloxy), benzyloxy, trialkyl-, triaryl- or tri-aralkyl-silyloxy (e.g. trimethylsilyloxy) with an anion or dianion of a lactam of general formula (V)

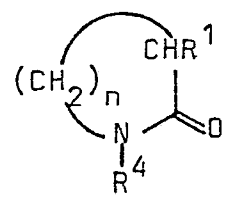

(V)

where n and $R^1$ are as defined above and $R^4$ is hydrogen, lower alkyl, aryl(lower)alkyl or trialkyl-, triaryl- or triarylalkyl-silyl (e.g. trimethylsilyl) (with the proviso that when $R^1$ is lower alkyl $R^4$ is other than hydrogen) and subjecting the product to hydrolysis. The lactam of general formula (V) can form its dianion when $R^4$ is hydrogen and an anion when $R^4$ is other than hydrogen. For example when $R^1$ is hydrogen and $R^4$ is other than hydrogen the anion of the lactam of general formula (V) may be formed *in situ* by reacting the lactam with an alkyl lithium (e.g. tertiary butyl lithium) or with a compound of general formula MA [where M is —MgX (where X is chlorine, bromine or iodine), sodium, potassium or lithium and A is a secondary amine radical]. When M is sodium, potassium or lithium the compound MA is a metal amide and is itself preferably formed *in situ* by reacting a metal compound $MR^5$ (where M is sodium potassium or lithium and $R^5$ is alkyl, aryl or aralkyl) with a secondary amine. The secondary amine may be a dialkylamine, e.g. diethylamine, di-isopropylamine, di-tertiarybutylamine, di-*n*-decylamine, dicyclohexylamine, N-*t*-amyl-N-*t*-butylamine, N-isopropyl-N-cyclohexylamine or N-(1'-ethylcyclohexyl)-1,1,3,3-tetramethylbutylamine or a cyclic compound, e.g. piperidine or 2,2,6,6-tetramethylpiperidine. A preferred metal amide is lithium diisopropylamine. When $R^1$ in the lactam of general formula (V) is lower alkyl or hydrogen and $R^4$ is other than hydrogen, the anion may be prepared by reacting the lactam with a Grignard reagent (preferably isopropylmagnesium bromide) or with a dialkylamino magnesium halide e.g. bromomagnesiumdiisopropylamide. When $R^4$ is hydrogen and $R^1$ is hydrogen the dianion of the lactam may be prepared by reacting the lactam with an alkyl lithium (e.g. tert.butyl lithium or butyl lithium) or with an alkali metal hydride (e.g. sodium hydride) followed by an alkyl lithium (e.g. butyl lithium).

The product of the reaction of the anion or dianion of the lactam of general formula (V) and the cyclohexene derivative (IV) is preferably not isolated but hydrolysed *in situ* to give the compound of general formula (I). If $R^4$ in the compound of general formula (V) is a tri-alkyl-, tri-aryl- or tri-aralkylsilyl group, this group is removed by the hydrolysis to give a compound of general formula (I) in which R is hydrogen.

The compounds of formula (I) and the derviatives of formula (II) are useful as intermediates for preparing pharmacologically active hexahydroazepine, piperidine and pyrrolidine derivatives as more fully described in European Patent Application No. 78300753.7 (Publication Number 0 003 253).

The following Examples illustrate the invention:

Example 1

Hexahydro-1-methyl-3-(3-oxocyclohexen-1-yl)-2*H*-azepin-2-one

Diisopropylamine (12.14 g, 17.0 ml) in dry tetrahydrofuran (THF 20 ml) was added dropwise to a stirred cooled (—10°C) solution of butyl lithium (86 ml of 1.4 molar solution in hexane) under nitrogen. After 10 minutes Gilman test was negative. 1-Methylcaprolactam (14.19 g) in THF (20 ml) was added, also at —10°C. The reaction mixture was stirred for 10 minutes at 0°C and 3-methoxy-2-cyclohexenone (12.6 g, 0.1 mole) in THF (20 ml) was added. The mixture was allowed to warm to room temperature and stirred at room temperature for 1.5 hours, then cooled to —10°C and decomposed by addition of 2N HCl (125 ml) rapidly but not allowing temperature for 30 minutes. The aqueous layer was separated and extracted with dichloromethane. The combined organic extracts were dried over anhydrous magnesium sulphate and evaporated to an oil which crystallised from toluene-light petroleum (b.p. 60—80°C) affording the title compound (18.99 g), m.p. 109—110°C.

| | | | |
|---|---|---|---|
| *Analysis*: Found: | C, 70.65; | H, 8.6; | N, 6.1% |
| $C_{13}H_{19}NO_2$ requires | C, 70.6; | H, 8.65; | N, 6.3%. |

Example 2

1-Methyl-3-(3-oxocyclohexen-1-yl)-2-piperidone

n-Butyllithium (1.4M in hexane, 120 ml) was treated at 20°C under dry nitrogen with diisopropylamine (27 ml, 19.2 g) in dry ether (25 ml). The mixture was stirred for 10 minutes at 20°C after completion of the addition and then 1-methyl-2-piperidone (20 g) in ether (25 ml) was added dropwise over 10 minutes. The mixture was stirred for a further 10 minutes, and then 3-isopropoxy-2-cyclohexenone (19.4 g) in ether (25 ml) was added dropwise over 10 minutes. The mixture was stirred at 20°C for a further 2 hours and was then hydrolysed by the addition, dropwise at first, of a mixture of conc. hydrochloric acid (50 ml) and (50 ml). The mixture was cooled in a water bath during the acidification to moderate the reaction. The mixture was cooled to room temperature (from 35°C) and the organic phase was separated and dried ($MgSO_4$). Removal of the solvent left a mobile yellow oil (0.63 g). The aqueous phase was extracted exhaustively with chloroform (10 × 40 ml) and the combined extracts were washed with water (100 ml), and saturated aqueous sodium chloride solution (100 ml), and dried ($MgSO_4$). Evaporation left a pale green oil (25.79 g). The two fractions were combined and distilled, giving two fractions A, bp < 120°C/1 mm (4.6 g), colourless, mobile liquid; and B, bp 150°C—164°C/0.07mm (14.46 g), yellow oil solidifying to a light yellow mass, mp 41—62°C. Fraction A was identified by IR as slightly impure 1-methyl-2-piperidone (23% recovery), while fraction B was identified by IR and NMR as the title compound.

Example 3

3-Ethyl-hexahydro-1-methyl-3-(3-oxocyclohexen-1-yl)-2H-azepin-2-one

A 2 molar solution of isopropylmagnesium bromide in ether (70 ml) was treated with 3-ethyl-hexahydro-1-methyl-2*H*-azepin-2-one (21.7 g, Aust. J. Chem. 1976, *29*, 2651) in THF (20 ml) and the mixture treated dropwise with diisopropylamine (19.6 ml) (exothermic). The reaction mixture was stirred for 2 hours then treated dropwise with 3-methoxy-2-cyclohexenone (12.6 g) in THF (20 ml). After stirring for 2 hours the reaction mixture was poured onto cold 2N HCl (250 ml). After 10 minutes the mixture was extracted with dichloromethane (2 × 300 ml), the combined organic phases washed with saturated aqueous $NaHCO_3$ solution and dried ($MgSO_4$). Removal of the solvents under reduced pressure followed by distillation gave the title compound as a viscous oil (Bpt 155—160°C/0.1mm) (13g). Redistillation (154—158°C/0.07mm) gave analytically pure material.

| | | | |
|---|---|---|---|
| *Analysis:* Found: | C, 72.4; | H, 9.6; | N, 5.4% |
| $C_{15}H_{23}NO_2$ requires | C, 72.25; | H, 9.3; | N, 5.6%. |

Example 4

Hexahydro-1-methyl-3-(3-oxocyclohexen-1-yl)-2*H*-azepin-2-one

2-Bromopropane (12.3 g) was added to a suspension of magnesium (2.43 g) in ether (50 ml) at such a rate to maintain gentle reflux and the mixture was stirred 30 minutes after addition was complete. Diisopropylamine (14 ml) was then added dropwise and the mixture stirred until the Gilman test showed negative (about 1 hour). N-methylcaprolactam (12.7 g) was added dropwise (exothermic). After addition of the N-methylcaprolactam, stirring became difficult due to separation of a sticky solid, which however redissolved on addition of THF (50 ml). After the addition was complete the reaction mixture was stirred for 30 minutes then treated dropwise with 3-isopropoxy-2-cyclohexenone (16.4 g) (exothermic) and stirred overnight. The reaction mixture was poured onto 2N aq HCl (250 ml) and stirred for 30 minutes. Dichloromethane (300 ml) was added and the layers separated. The aqueous layer was extracted with dichloromethane (2 × 300 ml) and the combined organic phases dried ($MgSO_4$). Removal of the solvents under reduced pressure followed by recrystallisation of the residue from ethyl acetate gave the title compound (13.2 g) identical with the product of Example 1.

Example 5

1-Methyl-3-(3-oxocyclohexen-1-yl)-2-pyrrolidone

n-Butyllithium (1.4 Molar in hexane, 190 ml) was treated dropwise over 10 minutes under dry nitrogen with diisopropylamine (30.3 g) in dry ether (50 ml), with external water cooling to maintain the reaction temperature below 25°C. After a further 10 minutes, freshly distilled dry 1-methyl-2-pyrrolidone (27.72 g) in dry ether (25 ml) was added dropwise over 15 minutes, and the suspension was stirred for a further 20 minutes at 20°C. 3-Isopropoxy-2-cyclohexenone (31 g) in ether (25 ml) was added to the mixture over 15 minutes, the suspended solid dissolving during the addition. The mixture was stirred for a further 2 hours at 20°C and was then cooled in ice and treated, dropwise at first, with a mixture of conc. hydrochloric acid (100 ml) and water (100 ml). After a further 10 minutes the phases were separated and the organic phase was discarded. The aqueous phase was extracted

with chloroform (10 × 50 ml) and the combined extracts were washed with water (100 ml) and saturated aqueous sodium chloride solution (100 ml) and dried ($MgSO_4$). Evaporation of the solvent gave an initially colourless oil which darkened in air to a clear red colour (37.82 g). Distillation of the oil gave the title compound as a pale yellow liquid which solidified on seeding to a yellow mass (32.75 g), bp 161°C/0.035 mm — 165°C/0.07 mm, m.p. 42—46°C.

Example 6

Hexahydro-3-(3-oxocyclohexen-1-yl)-2*H*-azepin-2-one

Lithium diisopropylamide, prepared by treating diisopropylamine (45.3 ml) with 1.4M butyl lithium in hexane (231 ml) at −10°C under nitrogen, was treated at −60°C with 1-trimethylsilylhexahydro-2*H*-azepin-2-one (63.7 g) in dry THF (50 ml). The white suspension was treated after 20 minutes with a solution of 3-methoxy-2-cyclohexenone (40.8 g) in THF (50 ml). The resulting solution was allowed to warm to ambient temperature. After a further 3 hours the cooled solution was treated with concentrated hydrochloric acid (120 ml) and stirred for 18 hours. The THF layer was combined with several chloroform extracts from the aqueous layer. Evaporation of the solvent gave a yellow solid, which was recrystallised from ethyl acetate to give hexahydro 3-(3-oxocyclohexen-1-yl)-2*H*-azepin-2-one as an off-white solid (45.5 g) m.p. 159—165°C.

| | | | |
|---|---|---|---|
| *Analysis*: Found: | C, 68.9; | H, 8.74; | N, 6.74%. |
| $C_{12}H_{17}NO_2$ requires | C, 69.54; | H, 8.27; | N, 6.76%. |

Example 7

Hexahydro-1-phenylmethyl-3-(3-oxocyclohexen-1-yl)-2*H*-azepin-2-one

A solution of hexahydro-1-phenylmethyl-2*H*-azepin-2-one (5.68 g) in dry THF was added at −10°C to lithium diisopropylamide (0.032 m) prepared from diisopropylamine (4.4 ml) and butyl lithium (22.9 ml) of 1.4M solution in hexane). The mixture was stirred for 30 minutes and then treated with a solution of 3-methoxy-2-cyclohexenone (2.53 g) in THF. After 5 hours at ambient temperature the mixture was poured into ice-cold concentrated hydrochloric acid (100 ml). After vigorously stirring for 12 hours the solution was shaken with several portions of chloroform. The combined chloroform layers were evaporated to give the title compound as an orange oil.

Example 8

Hexahydro-3-(3-oxocyclohexen-1-yl)-2*H*-azepin-2-one

Butyllithium (143 ml of 1.4 molar solution in hexane) was added dropwise to a stirred solution of caprolactam (11.3 g) in dry tetrahydrofuran under nitrogen. After stirring for 50 minutes at 0°, 3-methoxy-2-cyclohexenone (12.6 g) in tetrahydrofuran was added. After a further 30 minutes the reaction was poured onto 5M hydrochloric acid. The organic layer was separated and the aqueous layer was extracted with chloroform. The combined organic layers were dried over magnesium sulphate. Removal of the solvent under reduced pressure left 15 g of a yellow solid. The product was recrystallised from ethyl acetate affording 2.5 g of the title compound identical to that described in Example 6.

**Claims**

1. A compound of general formula (I)

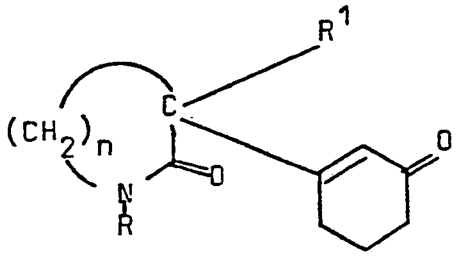

(I)

wherein n is 2, 3 or 4, R is hydrogen, lower alkyl or aryl(lower)alkyl and $R^1$ is hydrogen or lower alkyl (where the term "lower" means that the radical referred to contains 1 to 6 carbon atoms).

2. A compound as claimed in Claim 1 wherein n is 4.
3. Hexahydro-1-methyl-3-(3-oxocyclohexen-1-yl)-2*H*-azepin-2-one.
4. 1-Methyl-3-(3-oxocyclohexen-1-yl)-2-piperidone.
5. 3-Ethylhexahydro-1-methyl-3-(3-oxocyclohexen-1-yl)-2*H*-azepin-2-one.
6. 1-Methyl-3-(3-oxocyclohexen-1-yl)-2-pyrrolidone.
7. Hexahydro-3-(3-oxocyclohexen-1-yl)-2*H*-azepin-2-one.

8. Hexahydro-1-phenylmethyl-3-(3-oxocyclohexen-1-yl)-2*H*-azepin-2-one.

9. A process for preparing a compound claimed in Claim 1 which comprises reacting a cyclohexene derivative of general formula (IV)

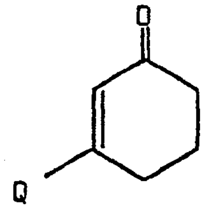

(IV)

where Q is a hydrolysable protecting group with an anion or dianion of a lactam of general formula (V)

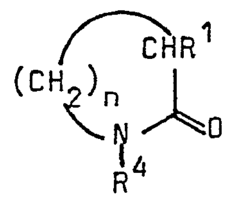

(V)

where n and $R^1$ are as defined in Claim 1 and $R^4$ is hydrogen, lower alkyl, aryl(lower)alkyl or trialkyl-, triaryl- or triarylalkyl-silyl (with the proviso that when $R^1$ is lower alkyl $R^4$ is other than hydrogen) and subjecting the product to hydrolysis; wherein the term "lower" means that the radical referred to contains 1 to 6 carbon atoms.

**Revendications**

1. Composé de formule générale (I)

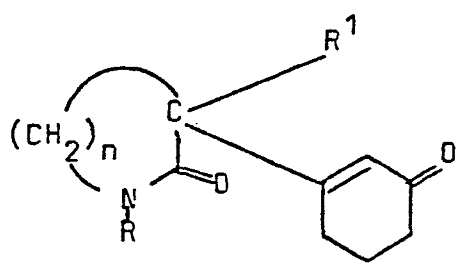

(I)

où n est 2, 3 ou 4, R est un atome d'hydrogène ou un radical alcoyle inférieur ou arylalcoyle inférieur et $R^1$ est un atome d'hydrogène ou un radical alcoyle inférieur (le terme "inférieur" signifiant que le radical concerné compte 1 à 6 atomes de carbone).

2. Composé suivant la revendication 1 dans lequel n est 4.

3. L'hexahydro-1-méthyl-3-(3-oxocyclohexène-1-yl)-2*H*-azépine-2-one.

4. La 1-méthyl-3-(3-oxocyclohexène-1-yl)-2-pipéridone.

5. La 3-éthylhexahydro-1-méthyl-3-(3-oxocycyclohexène-1-yl)-2*H*-azépine-2-one.

6. La 1-méthyl-3-(3-oxocyclohexène-1-yl)-2-pyrrolidone.

7. L'hexahydro-3-(3-oxocyclohexène-1-yl)-2*H*-azépine-2-one.

8. L'hexahydro-1-phénylméthyl-3-(3-oxocyclohexène-1-yl)-2*H*-azépine-2-one.

9. Procédé de préparation d'un composé suivant la revendication 1, qui comprend la réaction d'un dérivé de cyclohexène de formule générale (IV)

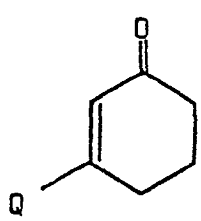

(IV)

où Q est un radical hydrolysable protecteur, avec un anion ou dianion d'un lactame de formule générale (V)

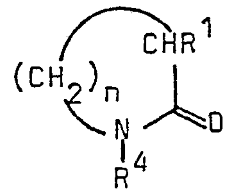

(IV)

où n et $R^1$ sont tels que définis dans la revendication 1 et $R^4$ est un atome d'hydrogène ou un radical alcoyle inférieur, arylalcoyle inférieur ou trialcoyl-, triaryl- ou triarylalcoylsilyle (avec la restriction que lorsque $R^1$ est un radical alcoyle inférieur, $R^4$ est autre qu'un atome d'hydrogène) et l'exécution d'une hydrolyse sur le produit; le terme "inférieur" signifiant que le radical concerné compte 1 à 6 atomes de carbone.

**Patentansprüche**

1. Verbindung der allgemeinen Formel (I)

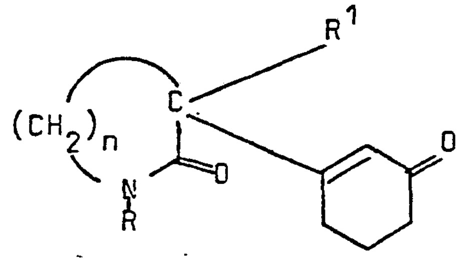

(I)

worin
n für 2, 3 oder 4 steht,
R Wasserstoff, Niedrigalkyl oder Aryl(niedrig)alkyl bedeutet und
$R^1$ für Wasserstoff oder Niedrigalkyl steht
(wobei die Bezeichnung "niedrig" bedeutet, daß der entsprechende Rest 1 bis 6 Kohlenstoffatome enthält).

2. Verbindung nach Anspruch 1, worin n für 4 steht.

3. Hexahydro-1-methyl-3-(3-oxocyclohexen-1-yl)-2*H*-azepin-2-on.

4. 1-Methyl-3-(3-oxocyclohexen-1-yl)-2-piperidon.

5. 3-Ethylhexahydro-1-methyl-3-(3-oxocyclohexen-1-yl)-2*H*-azepin-2-on.

6. 1-Methyl-3-(3-oxocyclohexen-1-yl)-2-pyrrolidon.

7. Hexahydro-3-(3-oxocyclohexen-1-yl)-2*H*-azepin-2-on.

8. Hexahydro-1-phenylmethyl-3-(3-oxocyclohexen-1-yl)-2*H*-azepin-2-on.

9. Verfahren zur Herstellung der Verbindungen nach Anspruch 1, wobei man ein cyclohexenderivat der allgemeinen Formel (IV)

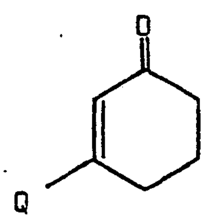

(IV)

worin
Q eine hydrolysierbare Schutzgruppe darstellt, mit einem Anion oder Dianion eines Lactams der allgemeinen Formel (V)

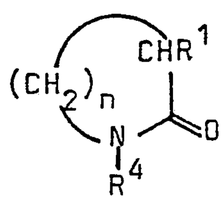

(V)

worin
n und $R^1$ die in Anspruch 1 angegebenen Bedeutungen besitzen
und
$R_4$ für Wasserstoff, Niedrigalkyl, Aryl(niedrig)alkyl oder Trialkyl-, Triaryl- oder Triarylalkyl-silyl

steht, (wobei Voraussetzung ist, daß wenn $R^1$ Niedrigalkyl bedeutet $R^4$ nicht Wasserstoff bedeuten kann)
umsetzt und das Produkt dann hydrolysiert;
wobei die Bezeichnung "Niedrig" bedeutet, daß der Rest 1 bis 6 Kohlenstoffatome bedeutet.